# EUROPÄISCHE PATENTSCHRIFT

(11) **EP 2 513 032 B1**
(45) Veröffentlichungstag und Bekanntmachung des Hinweises auf die Patenterteilung: **04.03.2015**
(21) Anmeldenummer: 10790771.9
(22) Anmeldetag: 13.12.2010
(51) Int. Cl.: C07C 67/62, C07C 57/075, C07C 69/54

(54) **VERFAHREN ZUR POLYMERISATIONSINHIBIERUNG VON (METH)ACRYLSÄURE UND/ODER (METH)ACRYLSÄUREESTERN**
METHOD FOR INHIBITING THE POLYMERIZATION OF (METH)ACRYLIC ACID AND/OR (METH)ACRYLIC ACID ESTERS
PROCÉDÉ DESTINÉ À INHIBER LA POLYMÉRISATION D'ACIDE (MÉTH)ACRYLIQUE ET/OU D'ESTERS D'ACIDE (MÉTH)ACRYLIQUE

(30) Priorität: 14.12.2009 US 286032 P; 14.12.2009 DE 102009058058
(43) Veröffentlichungstag der Anmeldung: 24.10.2012
(73) Patentinhaber: BASF SE, 67056 Ludwigshafen (DE)
(72) Erfinder: LIPOWSKY, Gunter, 68526 Ladenburg (DE); RISSEL, Steffen, 67281 Kirchheim (DE); SCHLIEPHAKE, Volker, 67105 Schifferstadt (DE); JÄGER, Ulrich, 67354 Römerberg (DE); HAREMZA, Sylke, 69151 Neckargemünd (DE)
(86) Internationale Anmeldenummer: PCT/EP2010/069440
(87) Internationale Veröffentlichungsnummer: WO 2011/082952

(56) Entgegenhaltungen:
- EP-A1- 1 688 407
- DE-A1-102005 030 416
- US-A1- 2006 151 309

## Beschreibung

Die vorliegende Erfindung betrifft ein Verfahren zur Polymerisationsinhibierung von (Meth)acrylsäure und/oder (Meth)acrylsäureester.

(Meth)acrylmonomere, insbesondere (Meth)acrylsäure und (Meth)acrylsäureester, sind wichtige Verbindungen zur Herstellung von Polymeren, die in verschiedensten Anwendungsbereichen (beispielsweise als superabsorbierende Polymere für Windeln, Dispersionen für Klebstoffe, Lacke, Farben, als Anstrichmittel und im Bereich der Textil- und Papierindustrie) eingesetzt werden.

Im Rahmen dieser Schrift sollen unter (Meth)acrylsäure sowohl Acrylsäure als auch Methacrylsäure, sowie unter (Meth)acrylsäureester sowohl Acrylsäureester als auch Methacrylsäureester verstanden werden.

Allgemein ist bekannt, dass polymerisationsfähige Verbindungen wie (Meth)acrylsäure und (Meth)acrylsäureester etwa durch Wärme oder Einwirkung von Licht oder Peroxiden leicht zur Polymerisation gebracht werden können. Es kommt jedoch besonders bei der Herstellung und Aufarbeitung immer wieder zu unerwünschter Polymerisation in verschiedenen Anlagenteilen, die eine Abstellung und Reinigung der Anlage erforderlich machen. Es besteht daher ein ständiger Bedarf an neuen, einfachen und wirtschaftlichen Methoden zur Verringerung der Polymerisation.

Die Polymerisationsinhibierung ist insbesondere dann von Bedeutung, wenn die polymerisationsfähige Verbindung in hoher Reinheit, beispielsweise eine Acrylsäure mit einem Reinheitsgrad von mind. 95 %, und im flüssigen Aggregatzustand vorliegt.

Es ist ein weit verbreiteter Stand der Technik, dass die Polymerisation von (Meth)acrylsäure und (Meth)acrylsäureestern durch die Verwendung von Polymerisationsinhibitoren, häufig in Verbindung mit sauerstoffhaltigen Gasen zurückgedrängt werden kann.

So offenbart die deutsche Offenlegungsschrift DE 19 746 689 A1 ein Verfahren zur Herstellung von (Meth)acrylsäure, bei dem Sauerstoff in Form von Luft in die Kolonne oder in den Verdampfer dosiert wird.

Aus der EP 1 035 102 A1 ist ein Verfahren zur Reinigung von (Meth)acrylsäure und (Meth)acrylsäureestern bekannt, wobei in die Leitungen zwischen Kolonnensumpf und Wärmetauscher ein sauerstoffhaltiges Gas dosiert wird.

In der WO 01/38285 A1 wird ein Verfahren zum Reinigen von (Meth)acrylmonomeren durch Destillation offenbart. Dabei wird eine Kombination eines in einer Flüssigkeit löslichen Polymerisationsinhibitors, wie beispielsweise Hydrochinonmonomethylether (MEHQ) oder Phenothiazin (PTZ), von Sauerstoff und NO₂ verwendet. Der Sauerstoff wird in Form von Luft in die Destillationsblase gegeben, NO₂ hingegen wird in die Zuführung und/oder in die für die Flüssigkeit nicht zugänglichen Stellen der Kolonne gegeben.

Ein Verfahren zur Aufarbeitung von (Meth)acrylsäure und (Meth)acrylsäureestern, bei dem ein sauerstoffhaltiges Gas eingeleitet wird, ist ebenfalls aus der DE 102 38 145 A1 bekannt. Das sauerstoffhaltige Gas wird zum Teil in den oberen Teil der Destillationskolonne sowie in den Sumpf der Kolonne dosiert.

Eine besondere Ausführungsvariante der Sauerstoffdosierung in die Destillationskolonne in einem Verfahren zur Herstellung von (Meth)acrylsäure und (Meth)acrylsäureestern offenbart die DE 103 39 633 A1. Dabei wird das sauerstoffhaltige Gas durch Öffnungen in Ringleitungen, die auf den jeweiligen Kolonnenböden installiert sind, mit einer Austrittsgeschwindigkeit von mindestens 50 mm/s eindosiert.

In der DE 102 56 147 A1 wird ein Verfahren der rektifikativen Auftrennung von (Meth)-acrylmonomeren enthaltenden Flüssigkeiten in einer Rektifikationskolonne beschrieben. Hierbei wird ein Stoffstrom der Kolonne entnommen und nach Behandlung mit Luft wenigstens ein Teilstrom dieses Stoffstroms als flüssige Phase wieder der Rektifikationskolonne zugeführt. Dabei ist der Sauerstoffgehalt des zurückgeführten Stroms mindestens doppelt so hoch wie der des entnommenen Stoffstroms. Die zur Behandlung des Stoffstroms eingesetzte Luft wird über einen Sättiger zugeführt. In dem erfindungsgemäßen Beispiel wird die Sättigung in einem zur Atmosphäre offenen Leerrohr mit Eindüsung der Acrylsäure aus einem Verweilzeitgefäß und vollständiger Rückführung zur Kolonnen beschrieben. Hierdurch wurde eine O₂-Sättigung von 45 ppm in Acrylsäure erreicht.

Aus der EP 1 084 740 A1 ist eine Vorrichtung bekannt, die sich zum Einleiten von sauerstoffhaltigen Gasen eignet. Die darin offenbarte Anordnung, die mit einem gewissen Abwärtswinkel an der Innenseite eines Apparates befestigt ist, verringert die Polymerisation.

Die Einleitung von sauerstoffhaltigen Gasen in den Sumpf der Kolonne bei der Herstellung von (Meth)acrylsäure und (Meth)acrylsäureestern ist ebenfalls aus den deutschen Offenlegungsschriften DE 2 362 373 und DE 2 202 980 sowie aus US 3,674.651 bekannt. Das sauerstoffhaltige Gas wird demnach in Kombination mit anderen Polymerisationsinhibitoren eingesetzt, wobei diese auch getrennt vom sauerstoffhaltigen Gas, beispielsweise über den Kolonnenkopf dosiert werden können.

Aus der DE 10 2005 030 416 A1 ist eine Anordnung zur Behandlung polymerisationsfähiger Stoffe bekannt, die zumindest eine Begasungsvorrichtung zum Einleiten eines Gases in den polymerisationsfähigen Stoff und eine Heizvorrichtung umfasst. Hierbei handelt es sich um senkrecht stehende Rohrbündelwärmetauscher, wobei die Flüssigkeit von oben nach unten fließt und die Luft im Gegenstrom von unten nach oben geführt wird.

Die sauerstoffhaltigen Gase werden im zitierten Stand der Technik bei Destillationsko-Ionnen oder bei Reaktoren in der Regel in den Sumpf, beispielsweise in den Sumpfumlauf, eindosiert. Diese Verfahren haben den Nachteil, dass sie die Polymerisation von (Meth)acrylsäure und (Meth)acrylsäureester nicht wirksam genug verhindern. Sie sind insbesondere nachteilig, wenn die (Meth)acrylsäure und/oder der (Meth)acrylsäureester bereits einen hohen Reinheitsgrad, beispielsweise von mindestens 95 %, aufweist und im flüssigen Aggregatzustand vorliegt.

Aus der EP 1 688 407 A1 ist ein Verfahren zum Reinigen von (Meth)acrylsäure bekannt, wobei Luft in einem Tank (sog. reflux tank), der mit (Meth)acrylsäuredestillationskondensat gefüllt wird, gegeben. Auf diese Weise wird die Stabilisierung durchgeführt. Die überschüssige Luft wird durch die Abgasleitung des Tanks entfernt.

Aus dem Stand der Technik ist jedoch kein Verfahren bekannt, das die Dosierung eines sauerstoffhaltigen Gases in eine (Meth)acrylsäure und/oder einen (Meth)acrylsäureester zwischen Produktion bzw. Aufarbeitung und Tankabfüllung offenbart, wobei die (Meth)acrylsäure und/oder der (Meth)acrylsäureester einen hohen Reinheitsgrad, beispielsweise von mindestens 95 %, aufweisen und im flüssigen Aggregatzustand vorliegen.

Aufgabe der vorliegenden Erfindung war es daher, ein Verfahren zur Polymerisationsinhibierung von (Meth)acrylsäure und (Meth)acrylsäureestern zur Verfügung zu stellen, mit dem deren Polymerisation wirksamer als mit den bisherigen Verfahren verringert werden kann. Das Verfahren sollte insbesondere dann gegen Polymerisationsinhibierung wirksam sein, wenn die (Meth)acrylsäure und/oder der (Meth)acrylsäureester bereits einen hohen Reinheitsgrad, beispielsweise von mindestens 95 %, aufweist und im flüssigen Aggregatzustand vorliegt.

Die Aufgabe wurde gelöst durch ein Verfahren zur Polymerisationsinhibierung von (Meth)acrylsäure und/oder (Meth)acrylsäureestern durch Einleiten eines sauerstoffhaltiges Gases in die (Meth)acrylsäure und/oder den (Meth)acrylsäureester, in dem die (Meth)acrylsäure und/oder der (Meth)acrylsäureester einen Reinheitsgrad von mindestens 95 % aufweist und im flüssigen Aggregatzustand vorliegt, wobei die Einleitung des sauerstoffhaltigen Gases in eine Leitung enthaltend flüssige (Meth)acrylsäure und/oder flüssigen (Meth)acrylsäureester, die als Reinprodukt nach der destillativen oder rektifikativen Aufreinigung in einer Kolonne durch einen Kopf- oder Seitenabzug einer Tankabfüllung zugeführt wird.

Erfindungswesentlich ist, dass das Verfahren zur Polymerisationsinhibierung dann Anwendung findet, wenn die (Meth)acrylsäure und/oder der (Meth)acrylsäureester bereits einen hohen Reinheitsgrad von mindestens 95 %. aufweist und im flüssigen Aggregatzustand vorliegt. Dies ist erfingdungsgemäß bei der Aufarbeitung von (Meth)acrylsäure und/oder (Meth)acrylsäureestern der Fall, die im Anschluss an eine destillative oder rektifikative Aufreinigung in einer Kolonne durch einen Seiten- oder Kopfabzug der Kolonne abgezogen, falls notwendig kondensiert und entweder der weiteren Aufarbeitung oder einer Abfüllung in beispielsweise einem Tank zugeführt werden.

Flüssige (Meth)acrylsäure und/oder flüssige (Meth)acrylsäureester mit einem dermaßen hohen Reinheitsgrad, sind besonders polymerisationsgefährdet. Obwohl diese (Meth)acrylsäure und/oder (Meth)acrylsäureester mit Polymerisationsinhibitoren, wie beispielsweise Hydrochinonmonomethylether oder Phenothiazin, sowohl während der Herstellung auch für die Lagerung versetzt werden, ist die Polymerisationsinhibierung nicht ausreichend.

Überraschenderweise wurde nun festgestellt, dass die Sauerstoffsättigung in der flüssigen (Meth)acrylsäure und/oder dem flüssigen (Meth)acrylsäureester zu gering ist, um In Kombination mit üblichen Polymerisationsinhibitoren für eine ausreichende Polymerisationsinhibierung zu bewirken. Dies ist insofern überraschend, als das während des gesamten Herstellungsprozesses und der Aufarbeitung bereits in Gegenwart von sauerstoffhaltigen Gasen gearbeitet wird. Daher wird erfindungsgemäß ein sauerstoffhaltiges Gas in die flüssige (Meth)acrylsäure oder den flüssigen (Meth)acrylsäureester geleitet, so dass für die Polymerisationsinhibierung ausreichend Sauerstoff in der flüssigen (Meth)acrylsäure oder dem flüssigen (Meth)acrylsäureester gelöst ist.

Die Herstellung und Aufarbeitung von (Meth)acrylsäure und (Meth)acrylsäureestern ist an sich bekannt. So wird die Herstellung von Acrylsäure beispielsweise in DE 103 39 633 A1, WO 02/090299 A2, WO 05/007610 A1 und WO 06/092410 A1 und der darin zitierten Literatur beschrieben. Aus den Schriften DE 101 44 490 A1, EP 0 733 617 A1, EP 1 081 125, DE 196 04 267 und DE 196 04 253 A1 und der darin zitierten Literatur ist u.a. die Herstellung von (Meth)acrylsäureestern bekannt.

Der Reinheitsgrad der (Meth)acrylsäure und/oder der (Meth)acrylsäureester beträgt dabei in der Regel mindestens 95 %, bevorzugt mindestens 97 %. Die genannten Verbindungen liegen im Seiten- oder Kopfabzug der Kolonne flüssig vor und müssen daher - falls erforderlich - mit einem geeigneten Kondensator kondensiert werden.

Eine Rein-Acrylsäure, die z.B. einer Tankabfüllung zugeführt wird, kann beispielsweise folgende Zusammensetzung aufweisen:

| | |
|---|---|
| Acrylsäure | 99,7 - 99,9 Gew.-% |
| Essigsäure | 50-1.500 Gew.-ppm |
| Propionsäure | 10 - 500 Gew.-ppm |
| Diacrylsäure | 50 - 1.000 Gew.-ppm |
| Wasser | 50 - 1.000 Gew.-ppm |
| Aldehyde und andere Carbonyle | 1 - 50 Gew.-ppm |
| Inhibitoren | 100 - 300 Gew.-ppm |
| Maleinsäure(-anhydrid) | 1 - 20 Gew.-ppm |

(Meth)acrylsäureester im Sinne der vorliegenden Erfindung können beispielsweise Methyl-, Ethyl-, n-Propyl-, iso-Propyl-, n-Butyl, sek-Butyl-, iso-Butyl-, tert.-Butyl-, Pentyl, Hexyl-, Octyl-, 2-Ethylhexyl-, 2-Propylheptyl-, Dodecyl-, 2-Hydroxyethyl-, 4-Hydroxybutyl-, 6-Hydroxyhexyl-, Dihydrocyclopentadienyl-, 2-Dimethylaminoethyl- oder Cyclohexyl-(meth)acrylat sowie Ethylenglykol-, 1,4-Butandiol- oder 1,6-Hexandioldi(meth)-acrylat, Trimethylolpropantriacrylat oder Pentaerythrittetraacrylat sein.

Selbstverständlich ist dieses Konzept auch auf andere polymerisationsfähige Verbindungen anwendbar, beispielsweise Styrol, Vinylacetat, Vinylpropionat, Allylessigsäure, Vinylessigsäure, N-Vinylformamid oder N-Vinylpyrrolidon.

Als sauerstoffhaltiges Gas kann reiner Sauerstoff, aber auch Gemische aus Sauerstoff und einem oder mehreren unter den Reaktionsbedingungen inerten Gasen in beliebigen Mischungen eingesetzt werden. Beispielsweise können Luft oder ein Gemisch aus Luft und einem unter den Reaktionsbedingungen inerten Gas verwendet werden. Als inertes Gas können Stickstoff, Helium, Argon, Kohlenmonoxid, Kohlendioxid, Wasserdampf, niedere Kohlenwasserstoffe oder deren Gemische verwendet werden. Der Sauerstoffgehalt des sauerstoffhaltigen Gases liegt zwischen 0,1 und 100 Vol-%, bevorzugt zwischen 1 und 50 Vol-% und besonders bevorzugt zwischen 3 und 21 Vol-%. Bevorzugt ist daher Luft oder ein Gemisch aus Luft mit einem unter den Reaktionsbedingungen inerten Gas.

Wie zuvor geschrieben, ist es erfindungswesentlich, dass die (Meth)acrylsäure oder der (Meth)acrylsäureester im flüssigen Aggregatzustand vorliegt. Daher liegt die Temperatur der (Meth)acrylsäure oder des (Meth)acrylsäureesters bei Atmosphährendruck in der Regel zwischen 0 und 100 °C, bevorzugt zwischen 20 und 90 °C und besonders bevorzugt zwischen 25 und 85 °C. Dies gilt selbstverständlich nur insoweit, als dass die (Meth)acrylsäure oder der (Meth)acrylsäureester bei den genannten Temperaturen und bei Atmosphärendruck auch im flüssigen Zustand vorliegen, d.h. der Schmelzpunkt und der Siedepunkt außerhalb des genannten Bereichs liegen.

Die Menge des eingespeisten Sauerstoffs in die flüssige (Meth)acrylsäure oder den flüssigen (Meth)acrylsäureester ist nicht beschränkt. Üblicherweise wird die Sauerstoffeinspeisung jedoch so eingestellt, dass der Sauerstoffgehalt in der flüssigen (Meth)acrylsäure oder dem flüssigen (Meth)acrylsäureester zwischen 0,3 und 56 ppm, bevorzugt zwischen 1 und 45 ppm und besonders bevorzugt zwischen 5 und 30 ppm beträgt.

Dabei ist die Sauerstoffsättigung in der flüssigen (Meth)acrylsäure oder dem flüssigen (Meth)acrylsäureester abhängig vom Sauerstoffgehalt des sauerstoffhaltigen Gases. In flüssiger Acrylsäure wird bei Einleiten von Luft (bei 20 °C unter Atmosphärendruck) eine 100 %-Sauerstoffsättigung erreicht, wenn die Acrylsäure einen Sauerstoffgehalt von 56 ppm aufweist; beim Einleiten von sog. Magerluft mit einem Sauerstoffgehalt von ca. 8 % wird eine 100 %-Sauerstoffsättigung bei einem Sauerstoffgehalt der flüssigen Acrylsäure von 25 ppm erreicht. Unter Verwendung von reinem Sauerstoff werden hingegen bis zu 280 ppm Sauerstoff in der flüssigen Acrylsäure gelöst.

Grundsätzlich ist es erforderlich, dass die Zufuhr des sauerstoffhaltigen Gases kontrolliert wird. Dazu muss zunächst ein Sensor zur Bestimmung des Sauerstoffgehalts in der flüssigen (Meth)acrylsäure oder des flüssigen (Meth)acrylsäureesters installiert sein, um daraus die noch erforderliche Menge an Sauerstoff bestimmen und einleiten zu können. Anschließend erfolgt die Kontrolle des eingeleiteten sauerstoffahltigen Gases dadurch, dass sämtliches zugeführtes sauerstoffhaltiges Gas in der flüssigen (Meth)acrylsäure oder dem flüssigen (Meth)acrylsäureester gelöst wird und sich keine Gasblasen bilden. Falls gewünscht, kann anschließend über einen zweiten Sensor nochmals der Sauerstoffgehalt der flüssigen (Meth)acrylsäure oder des flüssigen (Meth)acrylsäureesters bestimmt werden.

Darüber hinaus kann die Zufuhr des sauerstoffhaltigen Gases reguliert werden. Beispielsweise beträgt im Falle von Luft die Zufuhr zwischen 0,1 und 10 m³/h, bevorzugt zwischen 0,5 und 6 m³/h und besonders zwischen 0,5 und 2 m³/h. Die Zufuhr wird selbstverständlich dem Sauerstoffgehalt des sauerstoffhaltigen Gases angepasst.

Wie zuvor beschrieben, erfolgt die Zufuhr des sauerstoffhaltigen Gases an den Stellen im Prozess, an denen die (Meth)acrylsäure und/oder der (Meth)acrylsäureester einen hohen Reinheitsgrad von mindestens 95 % aufweist und im flüssigen Aggregatzustand vorliegen. Dies ist insbesondere bei der Aufarbeitung von (Meth)acrylsäure und/oder (Meth)acrylsäureestern der Fall, die im Anschluss an eine destillative oder rektifikative Aufreinigung in einer Kolonne durch einen Seiten- oder Kopfabzug der Kolonne abgezogen, falls notwendig kondensiert und entweder der weiteren Aufarbeitung oder einer Abfüllung in beispielsweise einem Tank zugeführt werden. Erfindungsgemäß erfolgt daher die Einleitung eines sauerstoffhaltigen Gases in eine Leitung enthaltend flüssige (Meth)acrylsäure und/oder flüssigen (Meth)acrylsäureester, die als Reinprodukt nach der destillativen oder rektifikativen Aufreinigung in einer Kolonne durch einen Kopf- oder Seitenabzug einer Tankabfüllung zugeführt wird.

Besonders bevorzugt eignet sich das erfindungsgemäße Verfahren für Acrylsäure als Reinprodukt. Diese wird üblicherweise als Reinsäure in einer Leitung mit einem Gefälle zum Tank zur Abfüllung geleitet.

Die Dosierung des sauerstoffhaltigen Gases kann an jeder möglichen Stelle der Leitung dosiert werden. Um jedoch auf die gesamte Strecke der Leitung eine ausreichende Polymerisationsinhibierung zu gewährleisten, erfolgt die Dosierung in Fliessrichtung der flüssigen (Meth)acrylsäure oder des flüssigen (Meth)acrylsäureesters bevorzugt in der vorderen Hälfte, besonders bevorzugt im vorderen Drittel der Leitung, durch die das Reinprodukt geführt wird.

Die Einspeisung des sauerstoffhaltigen Gases kann über beliebige Vorrichtungen erfolgen, die sich für das erfindungsgemäße Verfahren eignen und dem Fachmann bekannt sind.

Beispielsweise kann es sich um gebogene oder gerade Einsteck- oder Tauchrohre, die gegebenenfalls mit weiteren Öffnungen versehen sein können, Düsen oder Ventile, oder solche Vorrichtungen handeln, wie sie in der EP-A1 1 035102 beschrieben sind. Bevorzugt sind jedoch Einsteck- oder Tauchrohre, die gegebenenfalls mit weiteren Öffnungen versehen sind. Dabei sind die Vorrichtungen bevorzugt so angebracht, dass sie zumindest teilweise, bevorzugt vollständig mit den Öffnungen in die flüssige (Meth)acrylsäure oder den flüssigen (Meth)acrylsäureester eintauchen, um möglichst viel Sauerstoff im Reinprodukt zu lösen.

Das Material, aus dem die Dosierungsvorrichtungen gefertigt sind, ist erfindungsgemäß nicht entscheidend, es sollte bei den herrschenden Bedingungen gegenüber dem Reinprodukt korrosionsstabil sein. Bevorzugt sind sie aus Edelstahl oder Kupfer oder aus verkupfertem Material gefertigt, denkbar sind auch Kunststoffe, die unter den herrschenden Bedingungen stabil sind, wie z.B. Teflon^{®} oder Kevlar^{®}.

Bei den Öffnungen in den Vorrichtungen kann es sich beispielsweise um Löcher, Schlitze, Ventile oder Düsen handeln, bevorzugt um Löcher beliebiger Form, bevorzugt runde Löcher. Die Öffnungen können sich an beliebiger Stelle über die Dosierungsvorrichtungen verteilt befinden, beispielsweise auf der Unterseite und/oder an den Wandungen und/oder regellos über die Fläche der Dosierungsvorrichtungen verteilt, bevorzugt an der unteren Hälfte, besonders bevorzugt an der Unterseite. Selbstverständlich können die Vorrichtungen mehrere Öffnungen aufweisen, beispielsweise an der Unterseite und regellos über die Fläche der Dosiervorrichtungen verteilt.

Üblicherweise wird das Reinprodukt mit mindestens einem Stabilisator gegen Polymerisation stabilisiert. Dieser mindestens eine Stabilisator kann bereits im Reinprodukt nach der destillativen oder rektifikativen Aufarbeitung bereits enthalten sein, oder wird, wenn der Stabilisator durch die Aufreinigung teilweise oder vollständig abgetrennt wurde, dem Reinprodukt wieder zugesetzt werden.

Als Stabilisatoren sind beispielsweise geeignet phenolische Verbindungen, Amine, Nitroverbindungen, phosphor- oder schwefelhaltige Verbindungen, Hydroxylamine, N-Oxyle und bestimmte anorganische Salze, sowie gegebenenfalls Gemische davon.

Ganz besonders bevorzugt sind Phenothiazin, Hydrochinonmonomethylether und ein Gemisch aus Hydrochinonmonomethylether und Phenothiazin.

Falls die Zugabe von Stabilisator noch erforderlich ist, ist die Art der Zugabe des Stabilisators ist nicht beschränkt. Der zugesetzte Stabilisator kann jeweils einzeln oder als Gemisch zugesetzt werden, in flüssiger oder in in einem geeigneten Lösungsmittel gelöster Form, wobei das Lösungsmittel selber ein Stabilisator sein kann, wie z.B. in der deutschen Patentanmeldung DE 102 00 583 A1 beschrieben.

Wird ein Gemisch von mehreren Stabilisatoren eingesetzt, so können diese unabhängig voneinander an verschiedenen oder gleichen der vorgenannten Dosierstellen zugeführt werden. Wird ein Gemisch mehrerer Stabilisatoren eingesetzt, so können diese auch unabhängig voneinander in unterschiedlichen Lösungsmitteln gelöst werden.

Für eine ausreichende Polymerisationsinhibierung ist es erforderlich, dass die Konzentration des Stabilisators je Einzelsubstanz zwischen 1 und 10 000 ppm betragen kann, bevorzugt zwischen 10 und 5 000 ppm, besonders bevorzugt zwischen 30 und 1 000 ppm, ganz besonders bevorzugt zwischen 50 und 500 ppm und insbesondere zwischen 100 und 300 ppm liegt.

In dieser Schrift verwendete ppm- und Prozentangaben beziehen sich, falls nicht anders angegeben, auf Gewichtsprozente und -ppm.

US Provisional Patent Application No. 61/286,032, eingereicht am 14.12.2009 ist eingefügt in die vorliegende Anmeldung durch Literaturhinweis. Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

### Beispiele

Die Sauerstoffmessung wurde in allen Beispielen mit dem handelsüblichen Messgerät Hamllton Oxygold ausgestattet mit einem Knick-O₂-Meter durchgeführt.

### Vergleichsbeispiel 1

In einer großtechnischen Anlage zur Herstellung von Acrylsäure bestehend aus einer Rektifikationskolonne mit Trenneinbauten (wie in Beispiel 1 der DE 103 39 633 A1 beschrieben, Durchmesser 3,8 m, Länge 32 m) wurde im Seitenabzug eine flüssige Acrylsäure mit einer Reinheit von 99,6 % und einer Temperatur von 84 °C abgezogen. Die Sauerstoffmessung wurde nach ca. 50 m der Seitenabzugsleitung mit einem Teilstrom der flüssigen Acrylsäure in einem Umpumpkreislauf durchgeführt, wobei die Acrylsäure mittlerweile auf eine Temperatur von 35 °C abgekühlt war. Die Sauerstoffmessung wurde wie oben beschrieben durchgeführt. Es wurde kein Sauerstoff in der flüssigen Acrylsäure detektiert.

### Vergleichsbeispiel 2

In der im Beispiel 1 beschriebenen Anlage wurde in den Umpumpkreislauf aus zwei Leitungen Luft mit einer Zufuhr von je 1,5 m³/h in den Teilstrom der flüssigen Acrylsäure (T = 35 °C) eindosiert. Der mit Sauerstoff angereicherte Teilstrom der Acrylsäure wurde zum Beginn der Seitenabzugsleitung zurückgeführt und mit der flüssigen Acrylsäure, die unmittelbar aus der Rektifikationskolonne abgezogen wurde (T = 75 °C) vermischt. Anschließend wurde die Sauerstoffmessung wie in Beispiel 1 nach ca. 50 m der Seitenabzugsleitung durchgeführt. Der Sauerstoffanteil in der flüssigen Acrylsäure betrug 0,3 - 0,4 ppm.

### Beispiel

In der im Beispiel 1 beschriebenen Anlage wurde in die Seitenabzugsleitung Luft mit einer Zufuhr von 1 m³/h in die flüssige Acrylsäure (T = 75 °C) eindosiert. Die Sauerstoffmessung wurde wie in Beispiel 1 nach ca. 50 m der Seitenabzugsleitung durchgeführt. Der Sauerstoffanteil in der flüssigen Acrylsäure betrug 12 ppm.

### Vergleichsbeispiel 3

In der im Beispiel 1 beschriebenen Anlage wurde der Sauerstoff auf den Trennboden in Höhe der Seitenabzugsleitung dosiert. Die Sauerstoffmessung wurde wie in Beispiel 1 nach ca. 50 m der Seitenabzugsleitung durchgeführt. Es wurde kein Sauerstoff In der flüssigen Acrylsäure detektiert.

## Patentansprüche

1. Verfahren zur Polymerisationsinhibierung von (Meth)acrylsäure und/oder (Meth)acrylsäureestern durch Einleiten eines sauerstoffhaltiges Gases in die (Meth)acrylsäure und/oder den (Meth)acrylsäureester, wobei die (Meth)acrylsäure und/oder der (Meth)acrylsäureester einen Reinheitsgrad von mindestens 95 % aufweist und im flüssigen Aggregatzustand vorliegt, **dadurch gekennzeichnet, dass** die Einleitung des sauerstoffhaltigen Gases in eine Leitung enthaltend flüssige (Meth)acrylsäure und/oder flüssigen (Meth)acrylsäureester, die als Reinprodukt nach der destillativen oder rektifikativen Aufreinigung in einer Kolonne durch einen Kopf- oder Seitenabzug einer Tankabfüllung zugeführt wird.

2. Verfahren nach Anspruch 1, **dadurch gekennzeichnet, dass** der Sauerstoffgehalt des sauerstoffhaltigen Gases zwischen 0,1 und 100 Vol-% liegt

3. Verfahren nach Anspruch 1 oder 2, **dadurch gekennzeichnet, dass** der Sauerstoffgehalt des sauerstoffhaltigen Gases zwischen 1 und 50 Vol-% liegt

4. Verfahren nach einem der vorstehenden Ansprüche, **dadurch gekennzeichnet, dass** das sauerstoffhaltige Gas Luft oder ein Gemisch aus Luft mit einem unter den Reaktionsbedingungen inertem Gas ist.

5. Verfahren nach einem der vorstehenden Ansprüche, **dadurch gekennzeichnet, dass** der Sauerstoffgehalt durch das Einleiten des sauerstoffhaltigen Gases zwischen 0,3 und 56 ppm liegt.

6. Verfahren nach einem der vorstehenden Anspruche, **dadurch gekennzeichnet, dass** die Zufuhr von Luft zwischen 0,1 und 10 m³/ beträgt.

7. Verfahren nach einem der vorstehenden Ansprüche, **dadurch gekennzeichnet, dass** die Zufuhr von Luft zwischen 0,5 und 6 m³/h beträgt.

8. Verfahren nach einem der vorstehenden Ansprüche, **dadurch gekennzeichnet, dass** die Dosierung des sauerstoffhaltigen Gases in Fliessrichtung der flüssigen (Meth)acrylsäure oder des flüssigen (Meth)acrylsäureesters im vorderen Drittel der Leitung, durch die das Reinprodukt geführt wird, erfolgt.

## Claims

1. A process for inhibiting polymerization of (meth)acrylic acid and/or (meth)acrylic esters by introducing an oxygenous gas into the (meth)acrylic acid and/or the (meth)acrylic ester, the (meth)acrylic acid and/or the (meth)acrylic ester having a degree of purity of at least 95% and being in the liquid state, wherein the introduction of the oxygenous gas into a line comprising liquid (meth)acrylic acid and/or liquid (meth)acrylic ester which is supplied as a pure product to a tank dispenser through a top or side draw after distillative or rectificative purification in a column.

2. The process according to claim 1, wherein the oxygen content of the oxygenous gas is between 0.1 and 100% by volume.

3. The process according to claim 1 or 2, wherein the oxygen content of the oxygenous gas is between 1 and 50% by volume.

4. The process according to any of the preceding claims, wherein the oxygenous gas is air or a mixture of air with a gas which is inert under the reaction conditions.

5. The process according to any of the preceding claims, wherein the oxygen content is between 0.3 and 56 ppm by virtue of the introduction of the oxygenous gas.

6. The process according to any of the preceding claims, wherein the supply of air is between 0.1 and 10 m³/h.

7. The process according to any of the preceding claims, wherein the supply of air is between 0.5 and 6 m³/h.

8. The process according to any of the preceding claims, wherein the oxygenous gas is metered in in flow direction of the liquid (meth)acrylic acid or of the liquid (meth)acrylic ester in the front third of the line through which the pure product is conducted.

## Revendications

1. Procédé pour l'inhibition de la polymérisation d'acide (méth)acrylique et/ou d'ester de l'acide (méth)acrylique par introduction d'un gaz contenant de l'oxygène dans l'acide (méth)acrylique et/ou l'ester de l'acide (méth)acrylique, l'acide (méth)acrylique et/ou l'ester de l'acide (méth)acrylique présentant un degré de pureté d'au moins 95% et se trouvant dans un état d'agrégat liquide, **caractérisé en ce que** l'introduction du gaz contenant de l'oxygène dans une conduite contenant l'acide (méth)acrylique liquide et/ou l'ester liquide de l'acide (méth)acrylique, qui est introduit en tant que produit pur après la purification par distillation ou rectification dans une colonne via un soutirage en tête ou latéral dans un conditionnement en réservoir.

2. Procédé selon la revendication 1, **caractérisé en ce que** la teneur en oxygène du gaz contenant de l'oxygène se situe entre 0,1 et 100% en volume.

3. Procédé selon la revendication 1 ou 2, **caractérisé en ce que** la teneur en oxygène du gaz contenant de l'oxygène se situe entre 1 et 50% en volume.

4. Procédé selon l'une quelconque des revendications précédentes, **caractérisé en ce que** le gaz contenant de l'oxygène est de l'air ou un mélange d'air avec un gaz inerte dans les conditions de réaction.

5. Procédé selon l'une quelconque des revendications précédentes, **caractérisé en ce que** la teneur en oxygène, par l'introduction du gaz contenant de l'oxygène, se situe entre 0,3 et 56 ppm.

6. Procédé selon l'une quelconque des revendications précédentes, **caractérisé en ce que** l'alimentation en air se situe entre 0,1 et 10 m³/h.

7. Procédé selon l'une quelconque des revendications précédentes, **caractérisé en ce que** l'alimentation en air se situe entre 0,5 et 6 m³/h.

8. Procédé selon l'une quelconque des revendications précédentes, **caractérisé en ce que** le dosage du gaz contenant de l'oxygène a lieu dans le sens d'écoulement de l'acide (méth)acrylique liquide ou de l'ester liquide de l'acide (méth)acrylique dans le premier tiers de la conduite au travers de laquelle le produit pur est guidé.
